# EUROPEAN PATENT APPLICATION

(11) **EP 4 432 174 A1**
(43) Date of publication of application: **18.09.2024**
(21) Application number: 22900098.9
(22) Date of filing: 10.10.2022
(51) Int. Cl.: G06N 20/00

(54) **DATA PROCESSING METHOD AND DATA PROCESSING APPARATUS**

(30) Priority: 30.11.2021 CN 202111453147
(71) Applicant: Huawei Cloud Computing Technologies Co., Ltd., Gui'an New District, Guizhou 550025 (CN)
(72) Inventor: SHEN, Wen, Guizhou 550025 (CN); QIAO, Nan, Guizhou 550025 (CN); ZHANG, Lei, Guizhou 550025 (CN); TAO, Jianjun, Guizhou 550025 (CN)
(74) Representative: Grünecker Patent- und Rechtsanwälte PartG mbB
(86) International application number: PCT/CN2022/124247
(87) International publication number: WO 2023/098291

(57) **Abstract**

Embodiments of this application disclose a data processing method and a data processing apparatus, to improve prediction accuracy of an AI task model. The method in embodiments of this application includes: obtaining a plurality of types of data, where all of the plurality of types of data have different sources and different data types; performing knowledge extraction on the plurality of types of data to obtain a knowledge graph, where the knowledge graph includes a plurality of knowledge entities and an association relationship between the plurality of knowledge entities, and the plurality of knowledge entities include different data types; and performing knowledge representation on each knowledge entity by using a knowledge representation algorithm corresponding to a data type of each knowledge entity, and initializing a weight of the relationship between the plurality of knowledge entities in the knowledge graph, to obtain a vector graph, where the vector graph is used to train an artificial intelligence AI task model.

## Description

This application claims priority to Chinese Patent Application No. "202111453147.2", filed with the China National Intellectual Property Administration on November 30, 2021 and entitled "DATA PROCESSING METHOD AND DATA PROCESSING APPARATUS", which is incorporated herein by reference in its entirety.

### TECHNICAL FIELD

Embodiments of this application relate to the artificial intelligence field, and in particular, to a data processing method and a data processing apparatus.

### BACKGROUND

In recent years, related artificial intelligence (artificial intelligence, AI) technologies have been widely applied to various industries. A deep learning technology is an AI technology based on a deep neural network algorithm, and data is processed by simulating a working mechanism of a human brain. Currently, an AI model (for example, a deep learning model) is usually used to complete tasks in various application scenarios. The AI model may also be referred to as an AI task model.

In a current AI technology, the AI model needs to be trained by using a large amount of sample data. In some current technical solutions, the AI model is usually trained only by using sample data with a single data type. For example, when the AI technology is applied to a clinical decision support system (clinical decision support system, CDSS) in a medical field, sample data required for training of a deep learning-based disease diagnosis model in the CDSS usually only comes from an electronic medical record, and a type of the sample data is a text in the electronic medical record. Due to single sources and types of the sample data, prediction accuracy of the disease diagnosis model is low, and an effect of assisting in clinical decision-making is poor.

In some scenarios, the sample data used for AI model training may have different data sources and different data types. However, currently, when the sample data is used for the AI model training, because the sample data of the different sources and the different data types cannot be well represented, in a process of training the AI model, the AI model cannot learn a feature in the sample data. Consequently, task prediction accuracy of the AI task model obtained through training is low.

Therefore, how to represent sample data of different sources and different data types, so that an AI task model obtained through training by using the represented data is used to improve prediction accuracy of a task is a technical problem that needs to be urgently resolved currently.

### SUMMARY

Embodiments of this application provide a data processing method and a data processing apparatus, to improve prediction accuracy of an AI task model.

A first aspect of embodiments of this application provides a data processing method. The method is performed by a computer device, or may be performed by a component of the computer device, for example, a processor, a chip, or a chip system of the computer device, or may be implemented by a logical module or software that can implement all or some device functions. Using a computer device as an example, the data processing method includes: the computer device obtains a plurality of types of data, where all of the plurality of types of data have different data sources and different data types. The data sources are related to a type of a to-be-trained task, and include data generated by a person or data generated by a machine. The data types include a text, a numeric value, or an image. The computer device performs knowledge extraction on the plurality of types of data to obtain a knowledge graph, where the knowledge graph includes a plurality of knowledge entities and an association relationship between the plurality of knowledge entities. The knowledge entity includes key elements extracted from the plurality of types of data, and the plurality of knowledge entities include different data types. The computer device performs knowledge representation on each knowledge entity by using a knowledge representation algorithm corresponding to a data type of each knowledge entity, and initializes a weight of the relationship between the plurality of knowledge entities in the knowledge graph, to obtain a vector graph, where the vector graph is used to train an artificial intelligence AI task model.

In this embodiment of this application, sample data used by the computer device to train the AI task model is data having a plurality of sources and a plurality of types. In addition, the computer device represents an abstract knowledge graph as a vector graph that can be identified by the computer device by using knowledge representation algorithms corresponding to different data types. The computer device trains the AI task model based on a vector graph obtained by using the data having a plurality of sources and a plurality of types. This improves prediction accuracy of the AI task model.

In a possible implementation, in a process in which the computer device performs the knowledge extraction on the plurality of types of data to obtain the knowledge graph, the computer device performs the knowledge extraction on the plurality of types of data based on different knowledge levels, to obtain a knowledge graph of a plurality of knowledge levels. For example, when performing the knowledge extraction on a plurality of types of medical data to obtain a knowledge graph in a treatment field, the computer device may perform the knowledge extraction based on a plurality of knowledge levels such as a phenotypic level, a genetic level, or a metagenomic level, to obtain a knowledge graph having a plurality of knowledge levels associated with each other.

In this embodiment of this application, the knowledge graph obtained by the computer device is a knowledge graph having a plurality of knowledge levels associated with each other, and the AI task model is trained based on the knowledge graph of the plurality of knowledge levels. Because the knowledge graph involves the plurality of knowledge levels, coverage of the knowledge graph is improved, and prediction accuracy of the AI task model is further improved.

In a possible implementation, there is an association relationship between knowledge entities from different knowledge levels, and the association relationship is obtained from the plurality of types of data. For example, the computer device obtains a relationship between the knowledge entities through analysis based on semantic information of the plurality of types of data. Alternatively, the association relationship is obtained according to a preset rule. For example, the computer device pre-stores a knowledge association rule that is determined based on domain knowledge, and the computer device establishes association relationships between the knowledge entities at different levels according to the preset knowledge association rule.

In this embodiment of this application, the computer device obtains associations existing in the plurality of types of data, and establishes association relationships between knowledge entities at a same level or different levels according to the preset rule, to fully explore internal relationships between the knowledge entities at different knowledge levels. A plurality of methods for obtaining the association relationship fully explore the association relationships between the knowledge entities, and increase a data volume used for training the AI task model.

In a possible implementation, in a process in which the computer device performs the knowledge representation on each knowledge entity, the computer device determines, from a preset algorithm library based on a preset relationship and the data type of each knowledge entity, the knowledge representation algorithm corresponding to the data type of the knowledge entity. The computer device performs the knowledge representation on the knowledge entity based on the corresponding knowledge representation algorithm, to obtain a representation vector corresponding to the knowledge entity. For example, when the data type of the knowledge entity is a text type, the computer device selects a knowledge representation algorithm from the preset algorithm library based on a preset relationship between the text type and the knowledge representation algorithm. The knowledge representation algorithm corresponding to the text type is, for example, a knowledge graph embedding (knowledge graph embedding, KGE) algorithm, a bidirectional encoder representations from transformers (bidirectional encoder representations from transformers, BERT) algorithm, or a word2vec (word2vec) algorithm.

In this embodiment of this application, the computer device selects a corresponding knowledge representation algorithm from the preset algorithm library based on the data type of the knowledge entity. This improves representation efficiency of the knowledge entity and the association relationship.

In a possible implementation, the computer device determines, based on the data type of each knowledge entity, the knowledge representation algorithm that corresponds to the data type and that is input by a user, and performs the knowledge representation on the knowledge entity based on the corresponding knowledge representation algorithm, to obtain a representation vector corresponding to the knowledge entity.

In this embodiment of this application, the knowledge representation algorithm may be a knowledge representation algorithm defined by the user, so that applicability of different knowledge representation algorithms is improved.

In a possible implementation, the AI task model is an AI model used for disease diagnosis, and the plurality of types of data include at least two types of the following data: medical record data, an image check report, a gene regulatory expression network, and a metabolic network.

The data processing method in this embodiment of this application may be applied to a medical field. A trained AI task model may be an AI task model used for disease diagnosis. The disease diagnosis model is trained by using sample data from a plurality of sources, so that diagnosis accuracy of the disease diagnosis model is improved.

In a possible implementation, the computer device trains the AI task model based on the vector graph to obtain a trained AI task model.

In this embodiment of this application, the computer device trains the AI task model by using the vector graph obtained after knowledge graph representation, so that implementability of AI task model training is improved.

In a possible implementation, in a process in which the computer device trains the AI task model based on the vector graph, the computer device updates a weight in the vector graph.

In this embodiment of this application, the computer device may continuously update the weight in the vector graph, so that accuracy of the trained AI task model is improved.

In a possible implementation, the computer device performs task prediction by using the trained AI task model, to obtain a prediction result, and identifies, based on the updated vector graph, a key knowledge entity and/or a key association relationship in a knowledge graph corresponding to the task prediction, to obtain an explainable knowledge graph.

In this embodiment of this application, the computer device may identify the key knowledge entity and/or the key association relationship in the knowledge graph applied to the task prediction, so that explainability of a model prediction result is improved.

In a possible implementation, the computer device outputs the explainable knowledge graph through a graphical user interface GUI.

In this embodiment of this application, the computer device outputs the explainable knowledge graph through the graphical user interface GUI, so that implementability of the solution is improved.

A second aspect of embodiments of this application provides a data processing apparatus. The data processing apparatus includes an interface unit and a processing unit. The interface unit is configured to obtain a plurality of types of data, where all of the plurality of types of data have different sources and different data types. The processing unit is configured to perform knowledge extraction on the plurality of types of data to obtain a knowledge graph. The knowledge graph includes a plurality of knowledge entities and an association relationship between the plurality of knowledge entities, and the plurality of knowledge entities include different data types. The processing unit is further configured to: perform knowledge representation on each knowledge entity by using a knowledge representation algorithm corresponding to a data type of each knowledge entity, and initialize a weight of the relationship between the plurality of knowledge entities in the knowledge graph, to obtain a vector graph, where the vector graph is used to train an artificial intelligence AI task model.

In a possible implementation, the processing unit is specifically configured to perform the knowledge extraction on the plurality of types of data based on different knowledge levels, to obtain a knowledge graph of a plurality of knowledge levels.

In a possible implementation, there is an association relationship between knowledge entities from different knowledge levels, and the association relationship is obtained from the plurality of types of data, or the association relationship is obtained according to a preset rule.

In a possible implementation, the processing unit is specifically configured to determine, from a preset algorithm library based on the data type of each knowledge entity and a preset relationship, the knowledge representation algorithm corresponding to the data type of the knowledge entity, and perform the knowledge representation on the knowledge entity based on the corresponding knowledge representation algorithm, to obtain a representation vector corresponding to the knowledge entity.

In a possible implementation, a knowledge representation algorithm that corresponds to the data type and that is input by a user is determined based on the data type of each knowledge entity, and the knowledge representation is performed on the knowledge entity based on the corresponding knowledge representation algorithm, to obtain the representation vector corresponding to the knowledge entity.

In a possible implementation, the AI task model is an AI model used for disease diagnosis, and the plurality of types of data include at least two types of the following data: medical record data, an image check report, a gene regulatory expression network, and a metabolic network.

In a possible implementation, the processing unit is further configured to train the AI task model based on the vector graph, to obtain a trained AI task model.

In a possible implementation, the processing unit is specifically configured to update a weight in the vector graph.

In a possible implementation, the processing unit is further configured to perform task prediction by using the trained AI task model, to obtain a prediction result, and identify, based on the updated vector graph, a key knowledge entity and/or a key association relationship in a knowledge graph corresponding to the task prediction, to obtain an explainable knowledge graph.

In a possible implementation, the processing unit is further configured to output the explainable knowledge graph through a graphical user interface GUI.

A third aspect of embodiments of this application provides a computer device. The computer device includes a processor, the processor is coupled to a memory, the memory is configured to store instructions. When the instructions are executed by the processor, the computer device is enabled to perform the method according to any one of the first aspect or the possible implementations of the first aspect.

A fourth aspect of embodiments of this application provides a computer-readable storage medium. The computer-readable storage medium stores instructions. When the instructions are executed, a computer is enabled to perform the method according to any one of the first aspect or the possible implementations of the first aspect.

A fifth aspect of embodiments of this application provides a computer program product. The computer program product includes instructions. When the instructions are executed, a computer is enabled to implement the method according to any one of the first aspect or the possible implementations of the first aspect.

It may be understood that, for beneficial effects that can be achieved by the data processing apparatus, the computer device, the computer-readable medium, the computer program product, or the like provided above, refer to beneficial effects in a corresponding method. Details are not described herein again.

### BRIEF DESCRIPTION OF DRAWINGS

FIG. 1 is a schematic diagram of a system architecture of a data processing method according to an embodiment of this application;
FIG. 2 is a schematic flowchart of a data processing method according to an embodiment of this application;
FIG. 3A and FIG. 3B are schematic diagrams of knowledge extraction according to an embodiment of this application;
FIG. 4 is a schematic diagram of knowledge representation according to an embodiment of this application;
FIG. 5 is a schematic diagram of establishing an AI task model according to an embodiment of this application;
FIG. 6 is a schematic diagram of a data processing effect according to an embodiment of this application;
FIG. 7 is a schematic diagram of a structure of a data processing apparatus according to an embodiment of this application; and
FIG. 8 is a schematic diagram of a structure of a computer device according to an embodiment of this application.

### DESCRIPTION OF EMBODIMENTS

Embodiments of this application provide a data processing method and a data processing apparatus, to improve accuracy of clinical decision-making.

In the specification, claims, and accompanying drawings of this application, terms "first", "second", "third", "fourth", and the like (if existent) are intended to distinguish between similar objects but do not necessarily indicate a specific order or sequence. It should be understood that the data termed in such a way are interchangeable in proper circumstances, so that embodiments described herein can be implemented in other orders than the order illustrated or described herein. In addition, the terms "include", "have", and any variants thereof are intended to cover a non-exclusive inclusion. For example, a process, method, system, product, or device that includes a series of steps or units is not necessarily limited to those clearly listed steps or units, but may include other steps or units that are not clearly listed or are inherent to the process, method, product, or device.

In embodiments of this application, the word such as "example" or "for example" is used to represent giving an example, an illustration, or a description. Any embodiment or design solution described as "example" or "for example" in embodiments of this application should not be explained as being more preferred or advantageous than another embodiment or design solution. To be precise, the word such as "example" or "for example" is intended to present a related concept in a specific manner.

In the following, some terms in this application are explained and described, to facilitate understanding of a person skilled in the art.

Deep learning (deep learning, DL) is a machine learning technology based on a deep neural network algorithm. A main feature of the deep learning is to process and analyze data through multiple nonlinear transformations. For example, the deep learning is applied in scenarios such as image recognition, speech recognition, natural language processing, and medical image data.

Graph deep learning (graph deep learning, GDL) applies various deep learning algorithms to graph structure data, such as graph neural networks or graph convolutional networks. A graph convolutional neural network (graph convolutional network, GCN) is a type of neural network method for implementing convolution on graph structure data. For example, convolution is implemented on graph structure data by using a method such as a Laplacian matrix or Fourier transform.

A gene regulatory network (gene regulatory network, GRN) is a network of interaction between DNA and protein. Gene activity is regulated by transcription factors that are bound with DNA. Most transcription factors are bound with a plurality of binding sites in a gene group. Therefore, all cells have a complex gene regulatory network. For example, human gene group encodes about 1,400 transcription factors that regulate expression of more than 20,000 human genes. Technologies of the gene regulatory network include binding site analysis methods such as ChIP-chip, ChIP-seq, or the like.

A metabolic network (metabolic network, ME) is a network of chemicals in living cells connected by biochemical reactions. The biochemical reaction is a conversion of one chemical into another through enzyme catalysis. Thus, all chemicals in cells are part of a complex biochemical reaction network. This network is known as a metabolic network.

An electronic medical record (electronic medical records, EMR) is an electronic patient record based on a computer system.

Real world data (real word data, RWD) is data obtained from sources other than traditional clinical trials. Data sources include, for example, large-scale simple clinical trials, clinical trials in actual medical care, prospective observational studies or registered studies, database analysis, case reports, health management reports, electronic health records, and the like.

A knowledge graph (knowledge graph) is a graph-based data structure that consists of nodes and edges. Each node is a knowledge entity, and each edge is an association relationship between knowledge entities. Knowledge entities can be things in a real world, such as names, genders, or symptoms. Association relationships are used to express specific relationships between different knowledge entities.

The foregoing describes some terms in embodiments of this application. The following describes, with reference to the accompanying drawings, a data processing method and a data processing apparatus provided in embodiments of this application.

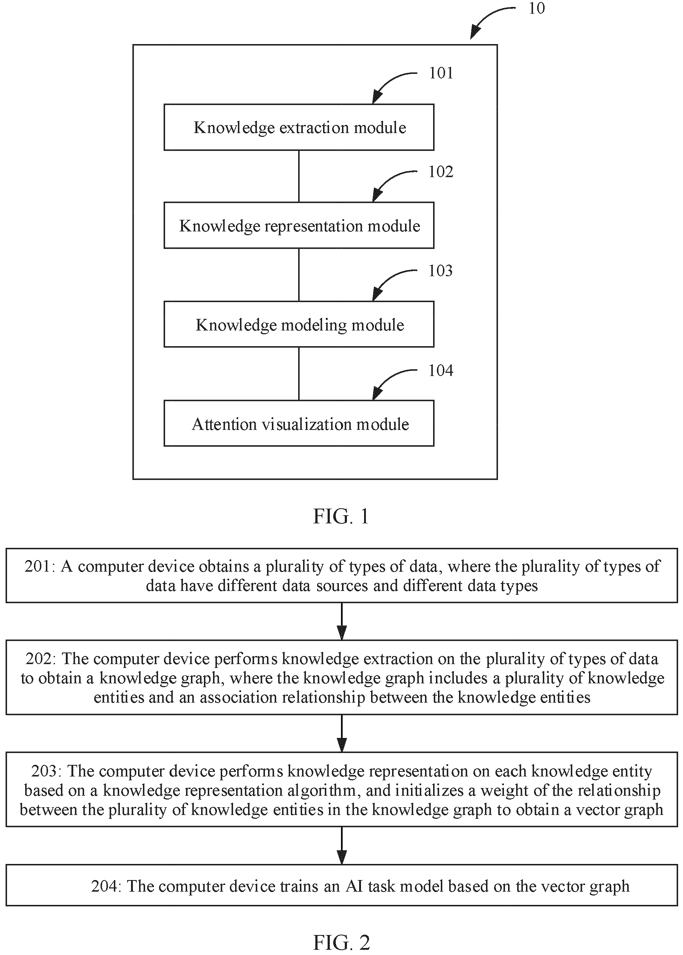

FIG. 1 is a schematic diagram of a system architecture of a data processing method according to an embodiment of this application. As shown in FIG. 1, a data processing system 10 includes a knowledge extraction module 101, a knowledge representation module 102, a knowledge modeling module 103, and an attention visualization module 104. Each module in the data processing system 10 may be independently invoked, and the data processing system 10 may also flexibly extend another module. This is not specifically limited.

It may be understood that each module of the data processing system 10 is a logical unit obtained through division based on system functions. An entity of the data processing system 10 may be a centralized or distributed computer device or server, or a component of a computer device or server, for example, a processor, a chip, or a chip system of a computer device.

The data processing system 10 in this embodiment of this application may integrate data based on related domain knowledge, train an AI task model, and predict an explainable result of a target event by using the AI task model. The data processing system 10 is a general framework for heterogeneous data processing and target event prediction, and may be applied to various fields. The following uses a medical field as an example to describe each module in the data processing system 10.

The knowledge extraction module 101 may extract available knowledge entities from data having a plurality of sources and a plurality of types, establish knowledge entities and at different knowledge levels based on data semantics or domain knowledge, and establish association relationships to form a knowledge graph. The knowledge graph contains knowledge entities and association relationships between the knowledge entities. The knowledge entities include element information extracted from a plurality of types of data, and the association relationships between the knowledge entities include relationships between the extracted element information. For example, in the medical field, a source of evaluation data of a physical status of a patient may be an electronic medical record, image data, a gene regulatory network, a protein metabolic network, or the like. The knowledge extraction module 101 may extract knowledge entities from the heterogeneous data of different sources and different data types, to form a knowledge graph with a connection characteristic. The knowledge entities are used as nodes in the knowledge graph, and association relationships between the knowledge entities are used as edges in the knowledge graph. The knowledge graph may extract key information in the heterogeneous data to a maximum extent.

Knowledge entity nodes in the knowledge graph may be at different knowledge levels, and association relationships between the knowledge entity nodes may also be association relationships across knowledge levels. For example, knowledge graphs at different knowledge levels include multi-level knowledge graphs such as a phenotypic level, a gene sequencing data level, a metabolic data level, or the like. There may be an association relationship between a knowledge entity in the knowledge graph at the phenotypic level and a knowledge entity at the gene sequencing data level.

The knowledge representation module 102 is configured to represent the knowledge graph by using a vector graph. The knowledge representation module 102 includes knowledge entities and association relationships between the knowledge entities in the knowledge graph. It may be understood that the knowledge graph obtained by the knowledge extraction module 101 cannot be directly used for AI task model training, and the knowledge representation module 102 needs to represent the knowledge entities in the knowledge graph as data in a vector form, and then train the AI task model by using the data. The knowledge representation module 102 includes a node module configured to represent the knowledge entities and an edge module configured to represent association relationships between the knowledge entities. A plurality of submodules are disposed in the node module and the edge module, and different submodules are configured to represent knowledge entities or association relationships of different data types.

The knowledge modeling module 103 is configured to obtain a deep learning model through training based on a vector graph. Deep learning models obtained through training based on different vector graphs support different downstream tasks. The deep learning model includes a graph convolutional network GCN, a graph attention network GAT, or a pattern book and an aggregated GraphSAGE. The deep learning model may further be integrated into a Transformer structure. Downstream tasks include auxiliary diagnosis tasks, check suggestion tasks, and drug recommendation tasks.

The attention visualization module 104 is configured to identify a key node and a key edge in the knowledge graph in the updated vector graph, and perform visual display, so that information about the key node and the key edge can be highlighted in the knowledge graph. The updated vector graph is a vector graph obtained after training of the deep learning model is completed.

FIG. 2 is a schematic flowchart of a data processing method according to an embodiment of this application. The method is applied to the data processing system shown in FIG. 1. For example, the data processing method is performed by a computer device. The data processing method includes the following steps.

201: The computer device obtains a plurality of types of data, where the plurality of types of data include data having different data sources and data types.

The computer device obtains the plurality of types of data, and the data are sample data for training an AI task model. The plurality of types of data obtained by a computing device have different data sources and data types. The data sources vary based on task types. Specifically, the plurality of types of data may be data generated by a person or data generated by a machine. The data types include a text, a numeric value, or an image.

In an example of a medical scenario, a computer obtains a plurality of medical data. The medical data may be real world data RWD, and the plurality of medical data have different data sources. For example, a plurality of sources of the medical data may be large-scale simple clinical trials, clinical trials in actual medical care, prospective observational studies, registered studies, retrospective database analysis, case reports, health management reports, medical record data, image check reports, gene regulatory expression networks, metabolic networks, proteomics information, or metagenomics information.

202: The computer device performs knowledge extraction on the plurality of types of data to obtain a knowledge graph, where the knowledge graph includes a plurality of knowledge entities and association relationships between the knowledge entities.

After obtaining the plurality of types of data, the computer device extracts, based on the obtained plurality of types of data, the knowledge entities and the association relationships between the knowledge entities. The knowledge entities and the association relationships between the knowledge entities form the knowledge graph.

Specifically, in a process of knowledge extraction, the computer device classifies extracted knowledge entities based on different knowledge levels, and establishes association relationships between the knowledge entities at the different knowledge levels. The knowledge entities at the different levels and the association relationships form a knowledge graph at a plurality of knowledge levels.

It should be noted that after obtaining the knowledge graph through extraction, the computer device needs to standardize the knowledge entities in the knowledge graph. For example, a knowledge entity extracted by the computer device from the electronic medical record is "stomachache", and a standardized knowledge entity is "abdominal pain".

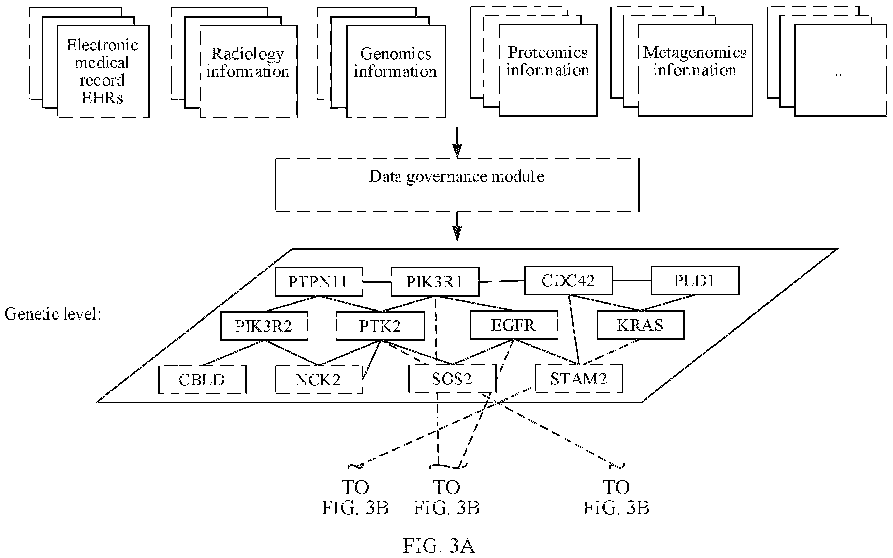

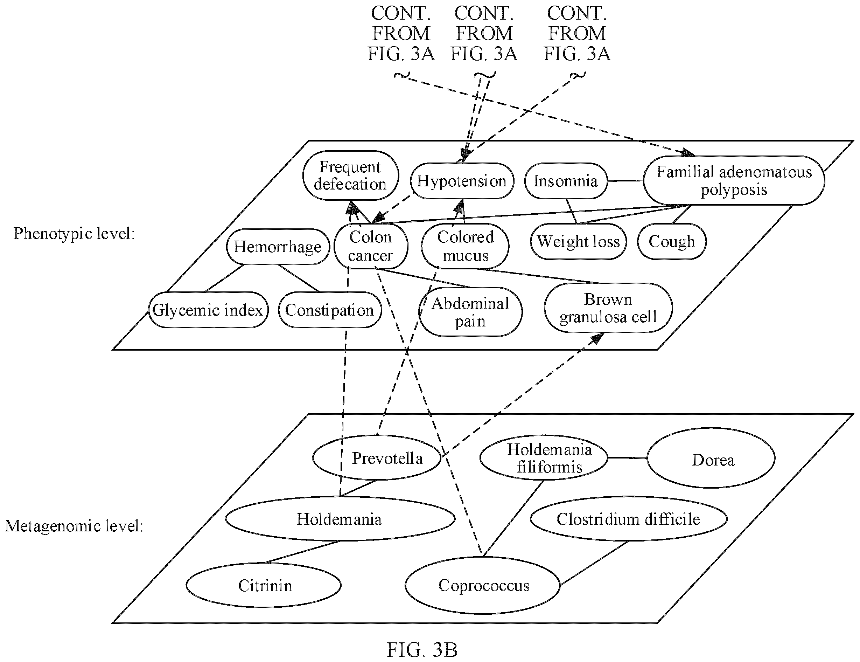

FIG. 3A and FIG. 3B are schematic diagrams of a knowledge graph at a plurality of knowledge levels in a medical field according to an embodiment of this application. In an example shown in FIG. 3A and FIG. 3B, the computer device performs knowledge extraction on a plurality of types of medical data, and the plurality of types of medical data include an electronic medical record, radiology information, genomics information, proteomics information, or metagenomics information. The computer device divides the extracted knowledge entities into a genetic level (genetic level), a phenotypic level (phenotypic level) and a metagenomic level (metagenomic level) based on different knowledge levels.

As shown in FIG. 3A and FIG. 3B, a knowledge entity at the genetic level is, for example, a PTPN11 gene, a PIK3R1 gene, or a CDC42 gene; a knowledge entity at the phenotypic level is, for example, frequent defecation, hypotension, or insomnia; and a knowledge entity at the metagenomic level is, for example, Prevotella, Holdemania filiformis, or Dorea.

In the example shown in FIG. 3A and FIG. 3B, after classifying the knowledge entities based on different knowledge levels, the computer device establishes the association relationships between the knowledge entities based on domain knowledge. The association relationships include association relationships between knowledge entities at a same knowledge level or association relationships between knowledge entities at different knowledge levels. In terms of the association relationships within the same knowledge level, for example, at the phenotypic level, there are association relationships between colon cancer and frequent defecation, abdominal pain and familial adenomatous polyposis (familial adenomatous polyposis, FAP). In terms of the association relationships within different knowledge levels, for example, there are association relationships between the hypotension at the phenotypic level and the PIK3R1 gene, an EGFR gene, and a KRAS gene at the genetic level.

In this embodiment of this application, the computer device may establish the association relationships between the knowledge entities based on the plurality of types of data, or may establish the association relationships between the knowledge entities according to a preset rule. The establishing the association relationships between the knowledge entities based on the plurality of types of data includes: The computer device analyzes semantics of the plurality of types of data, and explores associations included in the plurality of types of data. For example, an electronic medical record records that "a 42-year-old male patient has symptoms of drinking a lot of water, hyperglycemia, and frequent micturition". Knowledge entities extracted by the computer device based on electronic medical record data are "age 42, gender male, and symptoms of drinking a lot of water, hyperglycemia, and frequent micturition". Association relationships between these knowledge entities are established based on semantics.

The establishing the association relationships between the knowledge entities according to a preset rule includes: The computer device establishes the association relationships between the knowledge entities according to a rule formed by domain knowledge and experience. For example, a preset rule stored in a computing device is "Prevotella causes hypotension". When the knowledge entities extracted by the computer device are hypotension and Prevotella, the computer device establishes an association relationship between Prevotella and the hypotension.

In this embodiment of this application, the knowledge entities extracted by the computer device also include a plurality of data types. For example, types of the knowledge entities include a text or a numeric value. It should be noted that when the computer device in this embodiment of this application performs knowledge extraction on the plurality of types of data, there are some knowledge entities and association relationships that are not extracted by the computer device. The knowledge entities that are not extracted include hidden nodes that cannot be covered by the domain knowledge, and the association relationships that are not extracted include hidden association relationships that cannot be covered by the domain knowledge. Because the computer device cannot obtain these hidden nodes and hidden association relationships based on data semantics or the domain knowledge in a knowledge entity extraction process, the computer device establishes virtual knowledge nodes and virtual association relationships for these hidden nodes and hidden association relationships in a knowledge representation process. That is, a vector graph represented by the computer device includes the virtual knowledge nodes and the virtual association relationships that are not reflected in the knowledge graph.

For example, the knowledge graph obtained by the computer device includes two knowledge entities: "headache" and "cough", and no association relationship is established between the two knowledge entities. The computer device may add a virtual knowledge node, for example, an "impact factor 1", to the knowledge representation, and add a "hidden association 1" between the "impact factor 1" and the "headache" and a "hidden association 2" between the "impact factor 1" and the "cough". These virtual knowledge nodes and virtual association relationships do not exist in the extracted knowledge graph, but are reflected in the nodes and weights in the vector graph after representation.

203: The computer device performs knowledge representation on each knowledge entity based on a knowledge representation algorithm, and initializes a weight of the relationship between the plurality of knowledge entities in the knowledge graph to obtain a vector graph.

The computer device performs the knowledge representation on each knowledge entity based on the knowledge representation algorithm, and performs association representation on the association relationships between the knowledge entities, to obtain a vector graph corresponding to the knowledge graph.

Specifically, in a process of representing the knowledge entity, the computer device selects, based on a data type of the knowledge entity, a knowledge representation algorithm corresponding to the data type, and represents the knowledge entity by using the knowledge representation algorithm, to obtain a representation vector of the knowledge entity. The computer device represents the association relationship to obtain a representation vector of the association relationship. The representation vector of the knowledge entity and the representation vector of the association relationship form a vector graph, and the represented vector graph includes initialized weights between the plurality of knowledge entities.

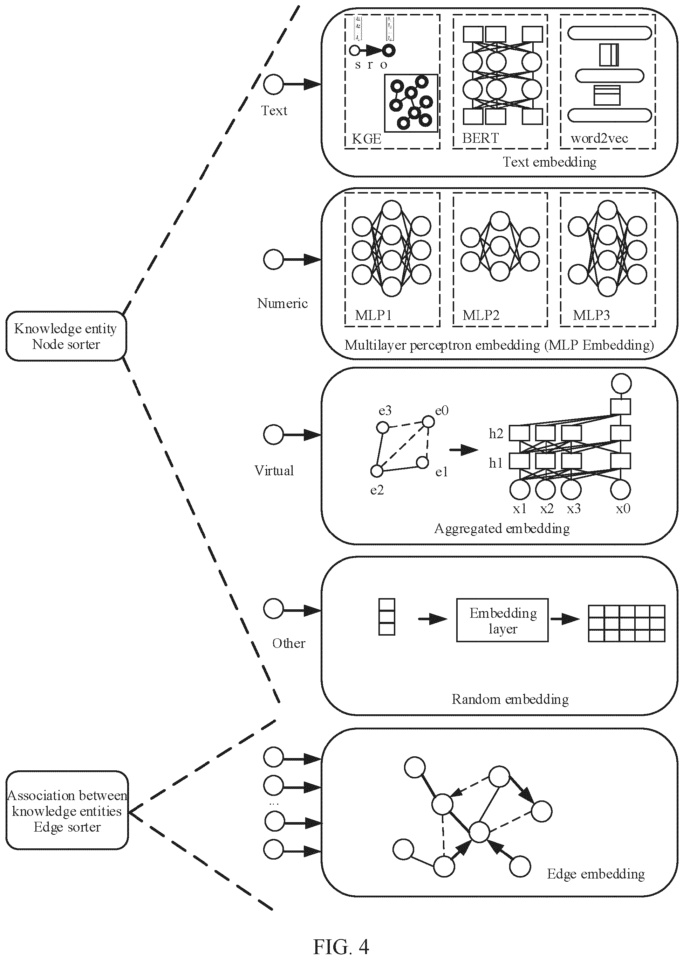

FIG. 4 is a schematic diagram of knowledge representation based on different data types according to an embodiment of this application. As shown in FIG. 4, knowledge entities are classified based on different data types. Types of the knowledge entities include text nodes, numeric value nodes, virtual nodes, and other nodes. A knowledge representation algorithm corresponding to the text nodes is, for example, a knowledge graph embedding (knowledge graph embedding, KGE) algorithm, a bidirectional encoder representations from transformers (bidirectional encoder representations from transformers, BERT) algorithm, or a word2vec (word2vec) algorithm. For example, the computer device obtains a representation vector of the text node by using the knowledge graph embedding algorithm. Specifically, the computer may first perform deep learning on an external source knowledge graph by using the knowledge graph embedding algorithm to obtain a representation vector of the external source knowledge graph, and first match the text nodes in the knowledge graph with knowledge entities in the external source knowledge graph, to obtain a representation vector of the knowledge graph. For another example, the computer device may alternatively pre-train a model based on a BERT algorithm in the medical field, and obtain a representation vector of the text node by using the model.

As shown in FIG. 4, a knowledge representation algorithm corresponding to a numeric value node is, for example, a multilayer perceptron (multilevel perceptron, MLP) algorithm. For example, the computer device classifies and encodes, based on an MLP model, a numeric value node such as a height, a weight, an age, or a check value, and maps the value node to a representation vector, to explore a meaning of data.

As shown in FIG. 4, for a virtual knowledge node, a computer device obtains a representation vector based on an aggregated embedding (aggregated embedding) algorithm, and for another node, the computer device obtains a representation vector based on a random embedding (random embedding) algorithm. In the example shown in FIG. 4, for association relationships between knowledge entities, the computer device obtains a representation vector of an edge by using an edge embedding algorithm (edge embedding).

The knowledge representation algorithm in this embodiment of this application may be a knowledge representation algorithm in a preset algorithm library, or may be a knowledge representation algorithm input by a user. This is not specifically limited. A one-to-one or many-to-one preset relationship exists between the knowledge representation algorithm and the data type in the preset algorithm library. The fixed data type includes a text or a numeric value. The knowledge representation algorithm input by the user is used to supplement the knowledge representation algorithm in the preset algorithm library.

The foregoing knowledge representation process is performed by a knowledge representation module in the computer device. The knowledge representation module may be flexibly decoupled in the computer device, may be adjusted or customized based on a field feature, and has scalability and interactivity. It may be understood that the knowledge representation module has different built-in representation submodules, and the representation submodules are configured to represent knowledge entities and association relationships of different data types.

204: The computer device trains the AI task model based on the vector graph.

The computer trains the AI task model based on the vector graph, and the trained AI task model may be used to execute various downstream tasks. The medical field is used as an example. The downstream tasks include medical consultation, drug recommendation, diagnosis decision support, treatment decision support, and the like.

Specifically, the computer device iteratively trains the AI task model based on the plurality of vector graphs obtained in the foregoing steps S201 to S203, until training output of the AI task model meets a deviation requirement between the training output and target output, the AI task model training is completed. In addition, the computer device obtains a vector graph dynamically updated based on a training process, and each node and weight in the updated vector graph are updated. When task prediction is performed by using the foregoing trained AI task model (for example, disease diagnosis is performed on a patient A based on a plurality of types of data of the patient A), the computer device represents to-be-predicted data in the manner of the foregoing steps S201 to S203, and obtains a prediction result by using a vector graph obtained after the representation and the trained AI task model.

The computer device further identifies, based on the updated vector graph obtained after the training, a key knowledge entity and/or a key association relationship in a knowledge graph corresponding to the task prediction, to obtain an explainable knowledge graph, and outputs the explainable knowledge graph through a graphical user interface. Specifically, after obtaining the updated vector graph, the computer device determines, based on weights of edges between nodes in the updated vector graph, an association relationship corresponding to an edge whose weight exceeds a preset threshold in the knowledge graph, and identifies, in the knowledge graph, the association relationship and the knowledge entity connected to the association.

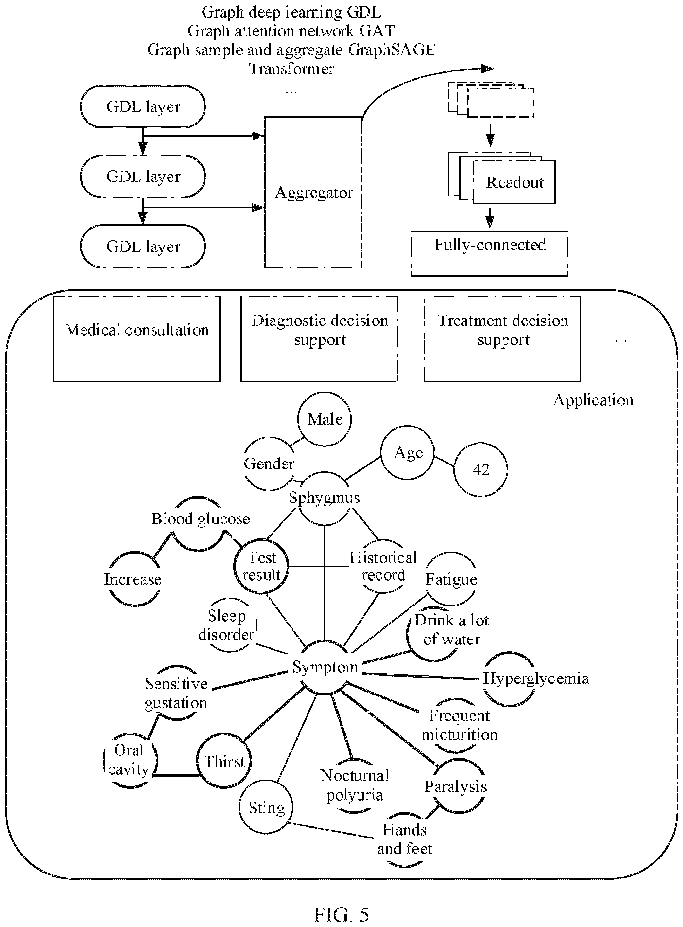

FIG. 5 is a schematic diagram of an explainable knowledge graph according to an embodiment of this application. As shown in FIG. 5, a knowledge graph shown in FIG. 5 is a knowledge graph corresponding to a disease diagnosis task. The computer device marks, based on a vector graph completed by an AI task model, a key node and a key edge in the knowledge graph corresponding to the task, to obtain an explainable knowledge graph. The explainable knowledge graph reflects a contribution degree of the key node and the key edge to the AI task model. The marking manner may be visually displayed in different colors and weight values based on the contribution degree, so that the key nodes and edge information in the graph can be highlighted. For example, in an example shown in FIG. 5, the node and the edge in bold are the key node and the key edge corresponding to the disease diagnosis task.

In embodiments of this application, an algorithm used by the computer device to train the AI task model includes a graph convolutional network (graph convolutional network, GCN), a graph attention network (graph attention network, GAT), or graph sample and aggregate (Graph sample and aggregate, GraphSAGE) training.

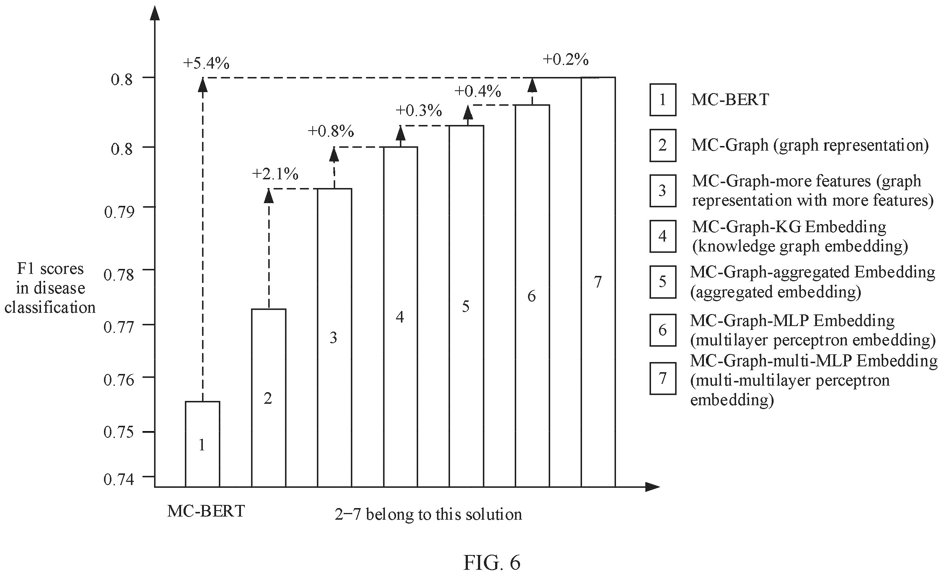

FIG. 6 is a schematic diagram of an F1 score of a data processing method according to an embodiment of this application. FIG. 6 is a diagram of comparison of F1 scores in a disease classification task in a data processing method according to an embodiment of this application. An F1 score is a harmonic average of a precision rate and a recall rate. The precision rate indicates a quantity of positive samples that are predicted to be positive, and the recall rate indicates a quantity of positive samples that are correctly predicted in the samples. The F1 score is used to evaluate classification accuracy of the disease classification task. A higher F1 score indicates more accurate disease classification. It can be learned from FIG. 6 that, the F1 score of a disease classification AI task model obtained through training based on a multi-dimensional graph embedding representation algorithm is increased by 5.7% at most in comparison with that of a disease classification AI task model obtained through training based on a BERT model direct text representation method.

In this embodiment of this application, sample data used by the computer device to train the AI task model is data having a plurality of sources and a plurality of types, and the AI task model is trained based on a knowledge graph extracted by using the data having the plurality of sources and the plurality of types, so that prediction accuracy of the AI task model is improved. In addition, the extracted knowledge graph includes knowledge entities and association relationships at a plurality of knowledge levels. This further improves prediction accuracy of the AI task model.

The foregoing describes a data processing method provided in embodiments of this application. The following describes a data processing apparatus in embodiments of this application with reference to the accompanying drawings.

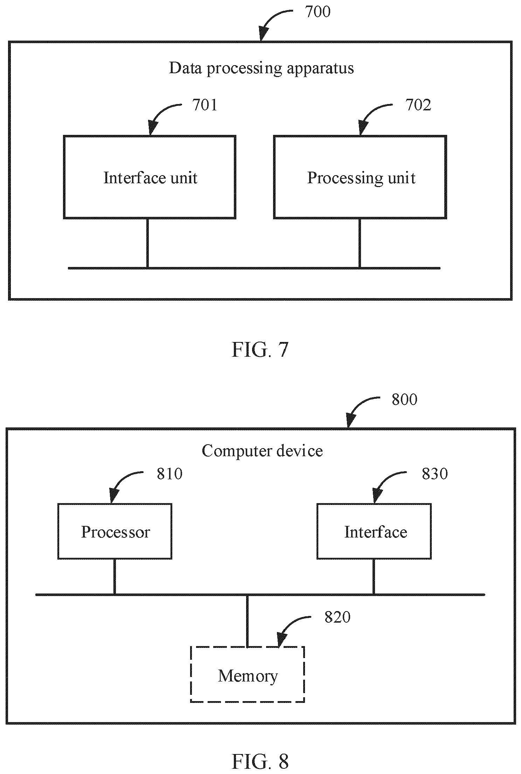

FIG. 7 is a schematic diagram of a structure of a data processing apparatus according to an embodiment of this application. The apparatus is configured to implement steps of corresponding devices in the foregoing embodiments. As shown in FIG. 7, the data processing apparatus 700 includes an interface unit 701 and a processing unit 702.

The interface unit 701 is configured to obtain a plurality of types of data, where all of the plurality of types of data have different sources and different data types. The processing unit 702 is configured to perform knowledge extraction on the plurality of types of data to obtain a knowledge graph. The knowledge graph includes a plurality of knowledge entities and an association relationship between the plurality of knowledge entities, and the plurality of knowledge entities include different data types. The processing unit 702 is further configured to: perform knowledge representation on each knowledge entity by using a knowledge representation algorithm corresponding to a data type of each knowledge entity, and initialize a weight of the relationship between the plurality of knowledge entities in the knowledge graph, to obtain a vector graph, where the vector graph is used to train an artificial intelligence AI task model.

In a possible implementation, the processing unit 702 is specifically configured to perform the knowledge extraction on the plurality of types of data based on different knowledge levels, to obtain a knowledge graph of a plurality of knowledge levels.

In a possible implementation, there is an association relationship between knowledge entities from different knowledge levels, and the association relationship is obtained from the plurality of types of data, or the association relationship is obtained according to a preset rule.

In a possible implementation, the processing unit 702 is specifically configured to determine, from a preset algorithm library based on the data type of each knowledge entity and a preset relationship, the knowledge representation algorithm corresponding to the data type of the knowledge entity, and perform the knowledge representation on the knowledge entity based on the corresponding knowledge representation algorithm, to obtain a representation vector corresponding to the knowledge entity.

In a possible implementation, a knowledge representation algorithm that corresponds to the data type and that is input by a user is determined based on the data type of each knowledge entity, and the knowledge representation is performed on the knowledge entity based on the corresponding knowledge representation algorithm, to obtain the representation vector corresponding to the knowledge entity.

In a possible implementation, the AI task model is an AI model used for disease diagnosis, and the plurality of types of data include at least two types of the following data: medical record data, an image check report, a gene regulatory expression network, and a metabolic network.

In a possible implementation, the processing unit 702 is further configured to train the AI task model based on the vector graph, to obtain a trained AI task model.

In a possible implementation, the processing unit 702 is specifically configured to update a weight in the vector graph.

In a possible implementation, the processing unit 702 is further configured to perform task prediction by using the trained AI task model, to obtain a prediction result, and identify, based on the updated vector graph, a key knowledge entity and/or a key association relationship in a knowledge graph corresponding to the task prediction, to obtain an explainable knowledge graph.

In a possible implementation, the processing unit 702 is further configured to output the explainable knowledge graph through a graphical user interface GUI.

It should be understood that division of units in the apparatus is merely logical function division. In actual implementation, all or some of the units may be integrated into one physical entity or may be physically separated. In addition, all the units in the apparatus may be implemented in a form in which a processing element invokes software, or may be implemented in a form of hardware; or some units may be implemented in a form in which a processing element invokes software, and some units are implemented in a form of hardware. For example, each unit may be a separately disposed processing element, or may be integrated into a chip of the apparatus for implementation. In addition, each unit may alternatively be stored in a memory in a form of a program to be invoked by a processing element of the apparatus to perform a function of the unit. In addition, all or some of the units may be integrated, or may be implemented independently. The processing element herein may also be referred to as a processor, and may be an integrated circuit having a signal processing capability. During implementation, steps in the foregoing methods or the foregoing units may be implemented by using an integrated logic circuit of hardware in a processor element, or may be implemented in a form in which a processing element invokes software.

It should be noted that, for ease of description, the foregoing method embodiments are described as a series of action combinations. However, a person skilled in the art should understand that the present invention and this application are not limited by the described action sequence. In addition, a person skilled in the art should also understand that embodiments described in this specification are all preferred embodiments, actions mentioned are not necessarily required for the present invention and this application.

Another appropriate step combination that can be figured out by a person skilled in the art according to the content described above also falls within the protection scope of the present invention and this application. In addition, a person skilled in the art should also be familiar to embodiments described in this specification all belong to preferred embodiments, and the related actions are not necessarily required by the present invention and this application.

FIG. 8 is a schematic diagram of a computer device according to an embodiment of this application. As shown in FIG. 8, the computer device 800 includes a processor 810, a memory 820, and an interface 830. The processor 810, the memory 820, and the interface 830 are coupled through a bus (not marked in the figure). The memory 820 stores instructions. When execution instructions in the memory 820 are executed, the computer device 800 performs the method performed by a first chip in the foregoing method embodiments.

The computer device 800 may be one or more integrated circuits configured to implement the foregoing methods, for example, one or more application specific integrated circuits (application specific integrated circuit, ASIC), one or more microprocessors (digital signal processor, DSP), one or more field programmable gate arrays (field programmable gate array, FPGA), or a combination of at least two of the integrated circuit forms. For another example, when the unit in the apparatus may be implemented in a form of scheduling a program by a processing element, the processing element may be a general-purpose processor, for example, a central processing unit (central processing unit, CPU) or another processor that can invoke the program. For still another example, the units may be integrated and implemented in a form of a system-on-a-chip (system-on-a-chip, SOC).

The processor 810 may be a central processing unit (central processing unit, CPU), or may be another general-purpose processor, a digital signal processor (digital signal processor, DSP), an application specific integrated circuit (application-specific integrated circuit, ASIC), a field programmable gate array (field programmable gate array, FPGA), or another programmable logic device, a transistor logic device, a hardware component, or any combination thereof. The general-purpose processor may be a microprocessor, or may be any conventional processor or the like.

The memory 820 may include a read-only memory and a random access memory, and provide instructions and data for the processor 810. The memory 820 may further include a non-volatile random access memory. For example, the memory 820 may be provided with a plurality of partitions, each area is used to store private keys of different software modules.

The memory 820 may be a volatile memory or a non-volatile memory, or may include both a volatile memory and a non-volatile memory. The non-volatile memory may be a read-only memory (read-only memory, ROM), a programmable read-only memory (programmable ROM, PROM), an erasable programmable read-only memory (erasable PROM, EPROM), an electrically erasable programmable read-only memory (electrically EPROM, EEPROM), or a flash memory. The volatile memory may be a random access memory (random access memory, RAM), and is used as an external cache. Through an example but not limitative description, many forms of RAMs may be used, for example, a static random access memory (static RAM, SRAM), a dynamic random access memory (DRAM), a synchronous dynamic random access memory (synchronous DRAM, SDRAM), a double data rate synchronous dynamic random access memory (double data rate SDRAM, DDR SDRAM), an enhanced synchronous dynamic random access memory (enhanced SDRAM, ESDRAM), a synchlink dynamic random access memory (synchlink DRAM, SLDRAM), and a direct rambus random access memory (direct rambus RAM, DR RAM).

In addition to a data bus, the bus may further include a power bus, a control bus, a status signal bus, and the like. The bus may be a peripheral component interconnect express (Peripheral Component Interconnect Express, PCIe) bus, an extended industry standard architecture (extended industry standard architecture, EISA) bus, a unified bus (unified bus, Ubus, or UB), a computer express link (computer express link, CXL), a cache coherent interconnect accelerators (cache coherent interconnect for accelerators, CCIX), or the like. The bus may be classified into an address bus, a data bus, a control bus, and the like.

According to another embodiment of this application, a computer-readable storage medium is further provided. The computer-readable storage medium stores computer-executable instructions. When a processor of a device executes the computer-executable instructions, the device performs the method performed by the computer device in the foregoing method embodiments.

According to another embodiment of this application, a computer program product is further provided. The computer program product includes computer-executable instructions, and the computer-executable instructions are stored in a computer-readable storage medium. When a processor of a device executes the computer-executable instructions, the device performs the method performed by the computer device in the foregoing method embodiments.

It may be clearly understood by a person skilled in the art that, for the purpose of convenient and brief description, for a detailed working process of the foregoing systems, apparatuses, and units, refer to a corresponding process in the foregoing method embodiments, and details are not described herein again.

In the several embodiments provided in this application, it should be understood that the disclosed systems, apparatuses, and methods may be implemented in other manners. For example, the described apparatus embodiments are merely examples. For example, division of the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, functional units in embodiments of this application may be integrated into one processing unit, each of the units may exist alone physically, or two or more units are integrated into one unit. The integrated unit may be implemented in a form of hardware, or may be implemented in a form of a software functional unit.

When the integrated unit is implemented in the form of the software functional unit and sold or used as an independent product, the integrated unit may be stored in a computer-readable storage medium. Based on such an understanding, the technical solutions of this application essentially, or the part contributing to a conventional technology, or all or some of the technical solutions may be implemented in the form of a software product. The computer software product is stored in a storage medium and includes several instructions for instructing a computer device (which may be a personal computer, a server, a network device, or the like) to perform all or some of the steps of the methods described in embodiments of this application. The foregoing storage medium includes any medium that can store program code, such as a USB flash drive, a removable hard disk, a read-only memory (ROM, read-only memory), a random access memory (RAM, random access memory), a magnetic disk, or an optical disc.

## Claims

1. A data processing method, comprising:
obtaining a plurality of types of data, wherein all of the plurality of types of data have different sources and different data types;
performing knowledge extraction on the plurality of types of data to obtain a knowledge graph, wherein the knowledge graph comprises a plurality of knowledge entities and an association relationship between the plurality of knowledge entities, and the plurality of knowledge entities comprise different data types; and
performing knowledge representation on each knowledge entity by using a knowledge representation algorithm corresponding to a data type of each knowledge entity, and initializing a weight of the association relationship between the plurality of knowledge entities in the knowledge graph, to obtain a vector graph, wherein the vector graph is used to train an artificial intelligence AI task model.

2. The method according to claim 1, wherein the performing knowledge extraction on the plurality of types of data to obtain a knowledge graph comprises:
performing the knowledge extraction on the plurality of types of data based on different knowledge levels, to obtain the knowledge graph of a plurality of knowledge levels.

3. The method according to claim 2, wherein there is an association relationship between knowledge entities from different knowledge levels, and the association relationship is obtained from the plurality of types of data, or the association relationship is obtained according to a preset rule.

4. The method according to any one of claims 1 to 3, wherein the performing knowledge representation on each knowledge entity by using a knowledge representation algorithm corresponding to a data type of each knowledge entity comprises:
determining, from a preset algorithm library based on the data type of each knowledge entity and a preset relationship, the knowledge representation algorithm corresponding to the data type of the knowledge entity, and performing the knowledge representation on the knowledge entity based on the corresponding knowledge representation algorithm, to obtain a representation vector corresponding to the knowledge entity; or
determining, based on the data type of each knowledge entity, the knowledge representation algorithm that corresponds to the data type and that is input by a user, and performing the knowledge representation on the knowledge entity based on the corresponding knowledge representation algorithm, to obtain a representation vector corresponding to the knowledge entity.

5. The method according to any one of claims 1 to 4, wherein the AI task model is an AI model used for disease diagnosis, and the plurality of types of data comprise at least two types of the following data: medical record data, an image check report, a gene regulatory expression network, and a metabolic network.

6. The method according to any one of claims 1 to 5, wherein the method further comprises:
training the AI task model based on the vector graph, to obtain a trained AI task model.

7. The method according to claim 6, wherein the training the AI task model based on the vector graph comprises: updating a weight in the vector graph.

8. The method according to claim 7, wherein the method further comprises: performing task prediction by using the trained AI task model, to obtain a prediction result; and
identifying, based on the updated vector graph, a key knowledge entity and/or a key association relationship in a knowledge graph corresponding to the task prediction, to obtain an explainable knowledge graph.

9. The method according to claim 8, wherein the method further comprises:
outputting the explainable knowledge graph through a graphical user interface GUI.

10. A data processing apparatus, comprising an interface unit and a processing unit, wherein
the interface unit is configured to obtain a plurality of types of data, wherein all of the plurality of types of data have different sources and different data types;
the processing unit is configured to perform knowledge extraction on the plurality of types of data to obtain a knowledge graph, wherein the knowledge graph comprises a plurality of knowledge entities and an association relationship between the plurality of knowledge entities, and the plurality of knowledge entities comprise different data types; and
the processing unit is further configured to: perform knowledge representation on each knowledge entity by using a knowledge representation algorithm corresponding to a data type of each knowledge entity, and initialize a weight of the association relationship between the plurality of knowledge entities in the knowledge graph, to obtain a vector graph, wherein the vector graph is used to train an artificial intelligence AI task model.

11. The apparatus according to claim 10, wherein the processing unit is specifically configured to:
perform the knowledge extraction on the plurality of types of data based on different knowledge levels, to obtain the knowledge graph of a plurality of knowledge levels.

12. The apparatus according to claim 11, wherein there is an association relationship between knowledge entities from different knowledge levels, and the association relationship is obtained from the plurality of types of data, or the association relationship is obtained according to a preset rule.

13. The apparatus according to any one of claims 10 to 12, wherein the processing unit is specifically configured to:
determine, from a preset algorithm library based on the data type of each knowledge entity and a preset relationship, the knowledge representation algorithm corresponding to the data type of the knowledge entity, and perform the knowledge representation on the knowledge entity based on the corresponding knowledge representation algorithm, to obtain a representation vector corresponding to the knowledge entity; or
determine, based on the data type of each knowledge entity, the knowledge representation algorithm that corresponds to the data type and that is input by a user, and perform the knowledge representation on the knowledge entity based on the corresponding knowledge representation algorithm, to obtain a representation vector corresponding to the knowledge entity.

14. The apparatus according to any one of claims 10 to 13, wherein the AI task model is an AI model used for disease diagnosis, and the plurality of types of data comprise at least two types of the following data: medical record data, an image check report, a gene regulatory expression network, and a metabolic network.

15. The apparatus according to any one of claims 10 to 14, wherein the processing unit is further configured to:
train the AI task model based on the vector graph, to obtain a trained AI task model.

16. The apparatus according to claim 15, wherein the processing unit is specifically configured to update a weight in the vector graph.

17. The apparatus according to claim 16, wherein the processing unit is further configured to: perform task prediction by using the trained AI task model, to obtain a prediction result; and
identify, based on the updated vector graph, a key knowledge entity and/or a key association relationship in a knowledge graph corresponding to the task prediction, to obtain an explainable knowledge graph.

18. The apparatus according to claim 17, wherein the processing unit is further configured to:
output the explainable knowledge graph through a graphical user interface GUI.

19. A computer device, comprising a processor, wherein the processor is coupled to a memory, the memory is configured to store instructions, and when the instructions are executed by the processor, the computer device is enabled to perform the method according to any one of claims 1 to 9.

20. A computer-readable storage medium, wherein the computer-readable storage medium stores instructions, and when the instructions are executed, a computer is enabled to perform the method according to any one of claims 1 to 9.

21. A computer program product, wherein the computer program product comprises instructions, and when the instructions are executed, a computer is enabled to implement the method according to any one of claims 1 to 9.
